# EUROPEAN PATENT APPLICATION

(11) **EP 2 703 018 A1**
(43) Date of publication of application: **05.03.2014**
(21) Application number: 12182569.9
(22) Date of filing: 31.08.2012
(51) Int. Cl.: A61L 27/52, A61L 27/56

(54) **One step method for casting hydrogels for tissue engineering**

(71) Applicant: Ludwig Boltzmann Gesellschaft GmbH, 1010 Vienna (AT)
(72) Inventor: Holnthoner, Wolfgang, 1080 Vienna (AT); Redl, Heinz, 1060 Vienna (AT)
(74) Representative: Sonn & Partner Patentanwälte

(57) **Abstract**

The present invention discloses a method for producing a hydrogel with one or more channels or a network of channels, said channels having a diameter of 1 cm to 50 µm, wherein one or more longitudinal members or a network of longitudinal members of a first material, said first material having a solubility in water at 25°C of 5 to 500 g/l, is provided and a hydrogel of a second material is formed which hydrogel covers and includes the longitudinal members at least partially and wherein the longitudinal member(s) is (are) at least partially removed from the hydrogel in the course of the formation of the hydrogel to form a hydrogel with one or more channels or a network of channels.

## Description

The present invention relates to methods for producing hydrogels for tissue engineering.

Tissue engineering has been an active field of research for several decades now. However, the amount of clinical applications in the field of tissue engineering is still limited. One of the current limitations of tissue engineering is its inability to provide sufficient blood supply in the initial phase after implantation. Insufficient vascularization can lead to improper cell integration or cell death in tissue-engineered constructs. For a tissue to grow beyond 100-200 µm (the diffusion limit of oxygen), new blood-vessel formation is required, and this is also true for tissue-engineered constructs. During in vitro culture, larger tissue-engineered constructs can be supplied with nutrients, for instance in perfusion bioreactors. However, after implantation of tissue constructs, the supply of oxygen and nutrients to the implant is often limited by diffusion processes that can only supply cells in proximity of 100-200 µm from the next capillary. In order for implanted tissues of greater size to survive, the tissue has to be vascularized, which means that a vascular network capable of delivering nutrients to the cells is formed within the tissue. After implantation, blood vessels from the host generally invade the tissue to form such a network, in part in response to signals that are secreted by the implanted cells as a reaction to hypoxia. However, this spontaneous vascular ingrowth is often limited to several tenths of micrometers per day, meaning that the time needed for complete vascularization of an implant of several millimeters is in the order of weeks. During this time, insufficient vascularization can lead to nutrient deficiencies and/or hypoxia deeper in the tissue. Moreover, nutrient and oxygen gradients will be present in the outer regions of the tissue, which could result in non-uniform cell differentiation and integration and thus decreased tissue function.

Therefore, larger tissue-engineered constructs require vascular structures to support cells with oxygen and nutrients. Recently, several strategies have been employed to prevascularise tissues, including different co-culture systems of endothelial cells and stem cells in various scaffolds in vitro and in vivo.

For example, functionalisation of different scaffolds, the inclusion of co-cultured cells, modular stacking techniques, provision of pores, direct encapsulation of cells in hydrogels, etc.; however all these techniques did not allow for directed construction of vascular networks.

To overcome this drawback, it was suggested to produce channels in the scaffold e.g. by 2-photon polymerization (Lee et al., 2008); micropattering (Moon et al., Tissue Eng. A 15 (2009), 579-585); solid free-form fabrication systems (Rouwkema et al., T. Biotech. 26 (2008); 434-441), incorporating phosphate-based glass fibres into collagen scaffolds to produce microchannels or by using a laser cutting system to provide holes into a scaffold (Lovett et al., Tissue Eng. B 15 (2009), 353-370; WO 2006/003442 A2); bioprinting (Norotte et al., Biomat. 30 (2009), 5910-5917); or by using microparticles (Behra et al., Macrom. Rap. Comm. 33 (2012), 1049-1054).

Major efforts were drawn to create scaffolds with microvascular structures (Lee et al., Biotech. Bioeng. 105 (2010), 1178-1186; Cui et al., Biomat. 30 (2009), 6221-6227; Chroback et al., Microvasc. Res. 71 (2006), 185-196) with channel diameters of below 50µm or even below 20 µm.

However, the scaffolds disclosed in the prior art have not been optimal: Either the scaffolds did not have a consistent formulation or were made from materials which were not applicable for tissue engineering; or were not suitable for vascularisation (microparticles) or did not allow sufficient vascularisation (pores); or resulted in random patterns (microparticles, pores, microvessels). Moreover, removal of the "channel placeholders" from such scaffolds to obtain the (micro-) channelled structure was difficult and often required the use of undesirable substances, such as organic solvents (e.g. dichloromethane) or separate steps (such as a heating step (e.g. Golden et al., Lab Chip 7 (2007), 720-725)).

Also the methods for providing the scaffolds according to the prior art have been either time consuming, cumbersome, requiring highly specific equipment (or undesired substances), etc. or - if the methods are easy to apply - are limited with respect to geometries, diameters of the channels, interconnection between the channels, control of geometries, etc. (reviewed in Huang et al., Biofab. 3 (2011) 012001).

It is therefore an object of the present invention to provide improved methods for producing scaffolds for tissue engineering. Such scaffolds should enable improved and defined structures for vascularisation and provide biocompatible and biodegradable structures suitable for tissue engineering, especially in human patients.

Therefore, the present invention provides a method for producing a hydrogel with one or more channels or a network of channels, said channels having a diameter of 1 cm to 50 µm, wherein one or more longitudinal members or a network of longitudinal members of a first material, said first material having a solubility in water at 25°C of 5 to 500 g/l, is provided and a hydrogel of a second material is formed which hydrogel covers and includes the longitudinal members at least partially and wherein the longitudinal member(s) is (are) at least partially removed from the hydrogel in the course of the formation of the hydrogel to form a hydrogel with one or more channels or a network of channels.

With the present invention, hydrogels are produced which contain a "man-made" channel structure which resemble natural vessel structures in tissues and allow an improved vascularisation after implantation. In the course of the present invention it was observed that suitable materials for tissue engineering do not necessarily have to provide microcapillaries. It turned out to be sufficient to provide macrovascular structures which can be infiltrated by cells in vivo or in vitro. The cells can then take care of creating microvessels by themselves ("cell-made") without the need to provide pre-casted capillaries in the scaffold. Accordingly, the present invention provides hydrogels with macrovascular structures (i.e. with channels of a diameter of 50 µm or more, preferably 100 µm or more, especially 150 µm or more) without microchannels. The method according to the present invention is simple but, nevertheless, allows provision of sophisticated, predefined vessel structures in the hydrogel scaffold. Further, due to its nature as a hydrogel, the three-dimensional structure is retained during and after production, in contrast to scaffolds which have been produced by applying pressure and layering. Since the channels are formed immediately during the casting process of the hydrogel by simple dissolution of the first material, the method according to the present invention provides a one-step casting process for the generation of hydrogels which are ready to use for tissue engineering without the need to apply a further step, such as heating or photocuring, or further "dissolving substances". In principle, any material can be used as "first material" which can be pre-formed to a suitable channel architecture in the dimensions from 50 µm upwards, and dissolves in the hydrogel water (or in the hydrogel buffer) in an appropriate time period. Of course, the hydrogel material has to have a solubility at the casting temperature which enabled hydrogel forming and simultaneous dissolution of the first material. The solubility of the first material has therefore to be suitable to initially forming the channels in the hydrogel casting process and dissolving after the initial formation during subsequent hydrogel finalisation.

According to the initial step of the method according to the present invention, a three-dimensional vascular structure is provided made of a first material which is then - later on in the casting process of the hydrogel - removed by simple dissolution in the hydrogel to provide the channel(s) which are supposed to be exogenously seeded by cells or directly transplanted into the patient. Provision of the three-dimensional structure is not dependent on any limitation with respect to the form, virtually any structure can be provided (as a "vessel blue-print"). Accordingly, only one or a few (2, 3, 4, 5, 6, 7, 8, 9 or 10) channels can be provided (connected (thereby creating a vessel network upon transplantation) or unconnected (thereby creating the possibility of parallel flow)) which form the predetermined architecture of the hydrogel. The number of interconnections is also flexible, depending on the intended use. Preferably, the hydrogel according to the present invention contains only one or a few interconnections between the longitudinal members, preferably 2, 3, 4, 5, 6, 7, 8, 9 or 10. The structure of the longitudinal member construct (which will become the channel architecture in the final hydrogel and the vessel structure in the implant) can be provided in any manner suitable for the first material chosen. For example, the longitudinal members can be pre-casted in preformed tubes (e.g. silicon tubes or a silicon tube system (which serves as a blueprint for the planned vessel architecture) or established by 3D printing.

The first material is chosen - depending on the second material (the hydrogel agent) - in a way that dissolution of the longitudinal members is possible during the casting process of the hydrogel and without negatively affecting the hydrogel with respect to its three-dimensional structure or its biocompatibility properties. Therefore, the first material has a solubility in water at 25°C of 5 to 500 g/l, preferably of 10 to 450, especially of 20 to 400. When the first material is removed by dissolution in the water/buffer of the hydrogel, channels are created which - in the end - will predetermine the vascular architecture of the implant. The first material therefore has to be a material which is stable during creation of the hydrogel (i.e. retains its three-dimensional structure when the hydrogel is casted around the one or more longitudinal members or network of longitudinal members of the first material) and is automatically removed after the hydrogel has initially established its three-dimensional structure at the beginning of the casting process. Although it is not possible to provide "man-made" microcapillary structures with the method according to the present invention, this turned out to be an advantage rather than a drawback of the present invention, since it turned out to be sufficient to provide macrovascular structures (diameters of 50 µm or more, preferably 100 µm or more) in the hydrogel. The microvascular structures needed are then established by the cells themselves ("cell-made") in an appropriate manner either in vitro (by providing the cells in the hydrogel) or in vitro (either by also providing the cells in the hydrogel or by invasion of cells from the patient). This allows the method of the present invention being excitingly practical, easy to use and easy to be established for industrial manufacturing purposes.

Since the method according to the present invention does not have to apply a separate step for dissolving the longitudinal members in the scaffold, not even a heating step, the method according to the present invention can be carried out at normal (room) temperatures without the need for heating, cooling or bringing in energy (e.g. by radiation, light, etc.). The method according to the present invention is therefore preferably carried out at a temperature of 5 to 40°C (at least: below 40°C), more preferred at 10 to 30°C (at least: below 30°C), especially 15 to 25°C (at least: below 25°C). A specific advantage of the method according to the present invention is that the whole casting process of the hydrogel (i.e. the initial formation of the hydrogel (wherein the three-dimensional structure is formed in principle) followed by the dissolution of the first material) can be carried out at constant temperature. Accordingly, the temperature of the casting process is preferably constant (but, of course, allows process-dependent temperature changes; nevertheless, the temperature should be preferably held constnt within ± 5°C, more preferred within ± 2°C, especially within ± 1°C.

According to the present invention, the first material is removed by dissolving the first material in the hydrogel.

According to the present invention the first material is automatically removed in the process for finalising the hydrogel structures. For enabling this, the first material only has to have a solubility in water that allows initial formation of the channels in the hydrogel and dissolves then in an acceptable amount of time. The (overall) casting process of the hydrogel should preferably be performable in a timeframe of 10 min to 24 h, more preferred of 20 min to 10 h, especially of 30 min to 5 h. Depending on the nature of the hydrogel material (made of the "second material"), the duration of the initial formation (the phase where the three-dimensional structure of the hydrogel is established (e.g. by polymerisation or swelling, etc.)) wherein the longitudinal members act as "placeholders" for the vessel structures of the final hydrogel may be rather short. Preferred duration of this initial formation (where dissolution processes of the first material should not occur or only occur in a limited amount (in order not to challenge the planned vessel structure of the final scaffold)) are 1 sec to 1 h, preferably 30 sec to 30 min. Then, the solubilisation process following the initial formation is slow enough to allow the hydrogel establishing its three-dimensional structure before the first material is dissolved upon contact with the water in the forming hydrogel. The channel-containing hydrogel according to the present invention is therefore finalised already with the finalisation of the hydrogel. A preferred embodiment of the present invention applies this principle: This embodiment uses polyethylene glycol (PEG)-based channels in hydrogels (especially fibrin hydrogels made of fibrinogen by treatment with thrombin), which self-dissolve during creation of the hydrogel.

Suitable examples of first materials are therefore easily degrading substances which can be provided as longitudinal members, such as PEGs, especially mixtures comprising low molecular weight PEGs, such as PEG 1000, or sugars, preferably saccharose.

PEG is a polymer which is synthesised out of ethylene oxide with a little amount of water to saturate the ends of the polymer chains. The reaction is a cationic polymerisation which uses tin (IV) chloride as catalyst. Depending on the degree of polymerisation this reaction delivers viscous or wax like products. PEG is non-toxic, water soluble and has a wide range of possible applications amongst others because of its multiple physical states and its chemical compatibility. In biomedicine PEG-based hydrogels function as matrices for controlled release of biomolecules and scaffolds for regenerative medicine. Such hydrogels are obtained by a variety of polymerisation techniques and can thereby be adjusted for the respective biomolecules. Generally, it was noticed that the higher the molecular weight of PEG the more brittle the resulting bar is, the lower the molecular weight the softer and more unstable it is. Using a mixture of PEGs of different molecular weight it is possible to combine the different properties. Using PEG as a first material, it is preferred to use a mixture of 5 to 50 % of a PEG with a molecular weight of about 1000 and 95 to 50 % of a PEG with a molecular weight of 2000 as a first material base for the longitudinal members according to the present invention. Specifically in connection with a fibrin hydrogel, this mixture has proven to provide excellent generation s of channels in the final hydrogel.

Hydrogels are determined as polymer networks that are insoluble in water, where they swell to an equilibrium volume but retain their shapes. The nature of the hydrogels used according to the present invention is not critical and mainly depends on the planned use and the nature of the first material (and the way in which the first material is removed from the hydrogel). The hydrogels used or suggested for use for tissue engineering and regenerative medicine are suitable, as they offer three-dimensional scaffolds to support the growth of cultured cells. In terms of material requirements, hydrogels have long received attention because of their innate structural and compositional similarities to the extracellular matrix and their extensive framework for cellular proliferation and survival. A variety of natural polymers, including agarose, collagen, fibrin, alginate, gelatin, chitosan and hyaluronic acid (HA), may preferably be used as hydrogel materials. These polymers are appealing for medical use owing to their similarity to the natural extracellular matrix (ECM), which allows cell adhesion, migration and proliferation, while maintaining very good biocompatible and biodegradable qualities.

Biocompatible macromolecules which have been used or suggested to be used in the creation of hydrogels for tissue engineering are polysaccharides (chondroitin sulfate (A type, B type, C type etc.), chitosan, heparin, gellan gum, arabic gum, xanthan gum, carrageenan, heparan, alginic acid, alginates, hyaluronic acid, agar, agarose, dermatan, dermatan sulfate, pectin, carboxymethyl cellulose, chitosan, etc.), their salt forms (e.g. sodium salt, potassium salt, etc.) and their chemical modified forms (e.g. carboxymethylation modification, hydrophobic modification, crosslinking, etc.), proteins (alkaline type gelatin, acidic type gelatin, alkaline type recombinant gelatin and acidic type recombinant gelatin, collagen, Matrigel, fibrin (made by fibrinogen degradation by thrombin), etc.) and their chemical modified forms (e.g. carboxylation modification and hydrophobic modification for amino group, etc.), and synthetic macromolecules (PEG, polyvinylpyrrolidone (PVP), polyacrylic acid, polyaspartic acid, polytartaric acid, polyglycolic acid, polylactic acid, polyglutamic acid, and polyfumaric acid, polyvinyl alcohol (PVA; hydrogels of PVA are preferably prepared by physically crosslinking an aqueous solution of PVA), poly 2-hydroxyethyl methacrylate (pHEMA) hydrogels, etc.) and their salt forms (e.g. sodium salt, potassium salt, etc.) and their chemical modified forms (e.g. carboxymethylation modification, hydrophobic modification, crosslinking, etc.).

In the recent past, a variety of very sophisticated hydrogels for tissue engineering purposes have been disclosed, such as hydrogels made from thermosensitive chitosan-beta-glycerol phosphate (GP) solutions, which undergo sol-gel transition around body temperature (chitosan 0.5-2% (w/v) mixed with GP 5-20% (w/v) solutions (Ahmadi et al., J Biomed Mater Res A. 86 (2008), 824-32); chemoenzymatically generated dextran-acrylate hydrogels (Ferreira et al., J Biomed Mater Res A. 68(2004), 584-96); antimicrobial hydrogel based on dimethyldecylammonium chitosan (with high quaternization)-graft-poly(ethylene glycol) methacrylate (DMDC-Q-g-EM) and poly(ethylene glycol) diacrylate (Li et al., Nature Materials 10 (2011), 149-156); dextran T40, methacrylated dextran (dex-MA) and hydroxyethyl-methacrylated dextran (dex-HEMA) (De Groot et al., Biomat. 22, (2001), 1197-1203); or polyacrylamide hydrogels (US RE38913). Accordingly, a preferred embodiment of the present invention applies a hydrogel which is formed of a gelation agent selected from fibrinogen, gelatin, chitosan, dextran, PEG, especially crosslinked PEG , polyacrylamide, hydroxypropyl methylcellulose, hydroxypropylcellulose, hydroxymethylcellulose, hydroxyethylcellulose, hydroxypropyl ethylcellulose, methylcellulose, ethylcellulose, carboxyethylcellulose, carboxymethyl hydroxyethylcellulose, carbomer, sodium carboxymethylcellulose, polyvinylpyrrolidone, or mixtures thereof, especially fibrinogen.

A specifically preferred hydrogel according to the present invention is a fibrin hydrogel. Fibrin is a naturally occurring product in the blood coagulation cascade and has been used for several years as a scaffold in tissue engineering, as it provides many important cellular adhesion molecules. It can be used as an autologous as well as homologous product. When fibrinogen, the precursor of fibrin, is mixed with thrombin, clot formation occurs, at a velocity which can be adapted by thrombin concentration variation. This clot can be resolved by proteases like plasmin or elastase and metalloproteinases. Moreover, the stability of the clot can be modified by the addition of protease inhibitors like aprotinin. Hence, fibrin is a suitable hydrogel scaffold for vascular tissue engineering purposes.

The exact dimensions of the longitudinal members of the first material is not critical, virtually any dimension is applicable according to the present invention which results in channels which can turn into vascular structures after implantation. For example, the longitudinal members can be provided with a diameter from 50 µm to 1 cm (larger vessels); however, the preferred diameter range of the longitudinal members according to the present invention are within 0.1 and 5 mm, especially within 0.2 to 1 mm. Preferred lengths of the longitudinal members are at least four times of the diameter, especially at least six times of the diameter, especially at least ten times of the diameter. Also networks of longitudinal members can be provided, for example with T- and/or Y-shaped connections (resulting in T-shaped or Y-shaped vessel connections in the implant) which can be pre-formed according to the necessities of the planned blood vessel structure for the implant.

According to a preferred embodiment, the hydrogel according to the present invention is provided with cells. A variety of cells have been used/suggested in tissue engineering. Preferably, endothelial cells or stem cells of human origin are used according to the present invention to improve vascularisation processes after implantation of the hydrogel. Also fibroblasts, especially dermal fibroblasts, represent preferred cells to be included in the present hydrogels. Seeding the hydrogel with human endothelial cells or human stem cells is specifically preferred for use of the present hydrogels for regenerative medicine. Via the adipose-derived stem cells (ASCs) in the surrounding hydrogel further microvascularisation of the construct ("cell-made") is induced, thereby ensuring oxygen and nutrient supply in larger tissues.

Since the hydrogels according to the present invention are preferably applied in humans, they have to be provided in sterilised form. Since preferred embodiments of the hydrogels contain cells which could be damaged by sterilisation procedures on the final products, it is preferred to apply sterilised components for the method according to the present invention and conduct the method under sterile conditions so as to arrive at a - still - sterile end product. Accordingly, it is preferred that the longitudinal members are sterilised before being provided as to cast the hydrogel. It is also preferred to use sterilised hydrogel forming material when forming the hydrogel. Since the hydrogel is usually casted by pouring a hydrogel forming solution or suspension over the longitudinal members, it is preferred to sterilise this solution or suspension before applying to the longitudinal members.

According to another aspect, the present invention relates to a hydrogel with one or more channels or a network of channels obtainable by a method according to the present invention.

Preferably, the hydrogel is sterile and preferably packaged in a pharmaceutically acceptable package. The hydrogel may be dried in this state (preferably without cells which would not survive such a drying step) and provided in a reconstitutable form, e.g. upon contacting the dried hydrogel with water (rehydrating) or a suitable reconstitution buffer. This might be advantageous in some circumstances because storage capabilities of the hydrogel are improved in the dried state.

According to a preferred embodiment, the hydrogel according to the present invention further contains cells for promoting vascularisation, preferably human stem cells, human endothelial cells, fibroblasts, especially dermal fibroblasts, or mixtures thereof. These cells could have been added already during production process of the hydrogel or immediately before administration to a patient.

The hydrogels according to the present invention can be provided in any form or dimensions currently available for other hydrogels used in tissue engineering, especially in regenerative medicine. Accordingly, the hydrogels according to the present invention have preferred volumes of 1 mm³ to 1 dm³, preferably of 0.1 cm³ to 50 cm³, especially 0.5 cm³ to 10 cm³. The shape of the hydrogel can also be predetermined according to the planned use and need of the individual indication or the individual patient.

The present hydrogels are preferably applied for inducing blood vessels in a human patient, especially in a patient with ischemic muscle, a peripheral vascular disease, myocardial infarction, ischemic chronic wounds or non-healing bone defects.

The invention is further described by the following examples and the drawing figures, yet without being restricted thereto.

Fig. 1 shows a PEG bar embedded in the Eppendorf tube lid (left panel), injection of black ink (right panel);

Fig. 2 shows a PEG-induced channels in fibrin clots filled with Microfil contrast agent (left panel); µCT picture of the channel (right panel);

Fig. 3 shows endothelial cells adhered to the PEG-generated channel in the fibrin matrix and are positive for CD31 (left panel), and negative for Caspase-3, indicating the absence of apoptotic cells, both shown by immunohistochemistry.

Fig. 4 shows various embodiments of the longitudinal members of the present hydrogels with different complexity, from a single member (4A) to a Y-shaped connected member (4B) to a more complex form with various interconnections (4C).

Fig. 5 shows cross sections of newly formed tube-like structures (arrowheads) 1 week after seeding with endothelial cells in the PEG-"made" channel (arrow); 200 x magnification.

Fig. 6 shows tube-like structure formation of OEC in co-culture with ASC in fibrin matrix (right panel; B). OEC alone did not form tube-like structures (left panel; A).

Fig. 7 shows thick or thin PEG threads, prepared to be used in a cap of an Eppendorf tube, were dissolved in 10mL NaCl 0.9% prewarmed to 37°C. A, B: 5 min dissolution time; C, D: 3 min dissolution time; immediately after putting the thread into the solution (A, C); immediately after dissolution (B, D).

Fig. 8 shows thick sugar threads were dissolved in 10mL NaCl 0.9% prewarmed to 37°C. A, B: 6 min dissolution time; C, D: 6 min dissolution time; immediately after putting the thread into the solution (A, C); immediately after dissolution (B, D).

### Examples:

### 1) Generation of the PEG structures:

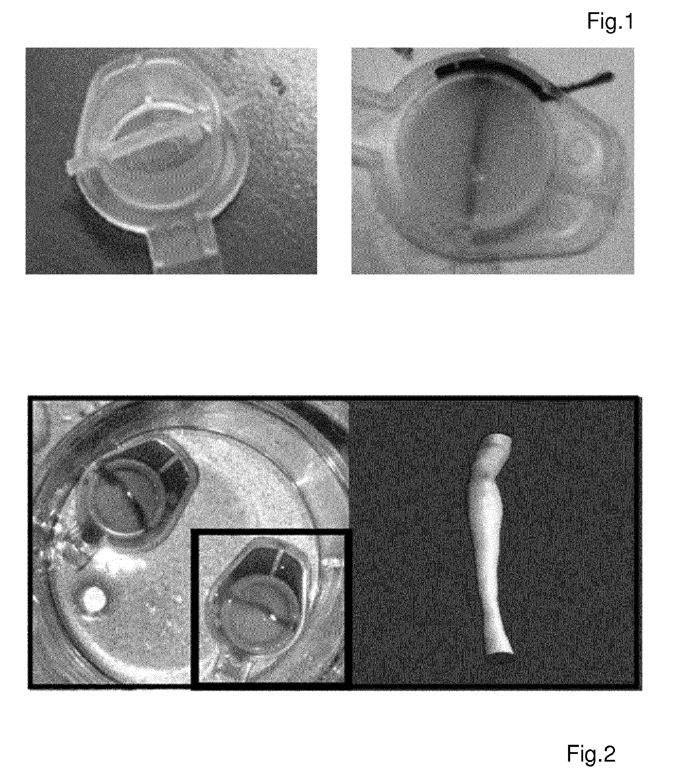

Pilot results showed that a mixture of 12.5 % PEG1000 and 87.5 % PEG2050 is well suited to generate the channel in the fibrin hydrogel. Thus, this mixture was generated and the resulting bar was embedded in the lid of an Eppendorf tube (Figure 1, left panel) .

Thereafter, the fibrin matrix (2.5 % end concentration) was poured around and the PEG bar was dissolved. To prove that the channel is functional, black ink was injected, and the channel became visible (Figure 1, right panel).

### 2) Structure and perfusion of the channel: 3D-visualisation by µCT

Fibrin clots (2.5 mg/ml fibrinogen) were casted on polyethylene glycol (PEG) bars. After 5 days incubation in EGM-2 supplemented with 100 KIE/ml aprotinin, Microfil contrast agent was injected into the channel (Figure 2, left panel). The µCT picture shows the channel after overnight polymerization of Microfil at 4°C (Figure 2, right panel).

### 3) Endothelial cell seeding into the fibrin channel

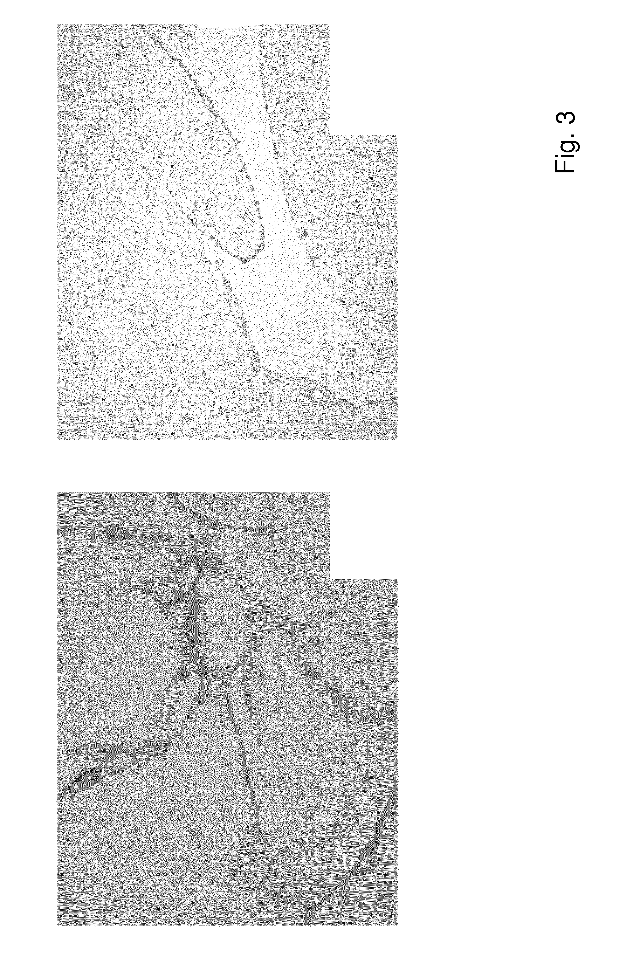

PEG bars (12.5 % PEG1000, 87.5 % PEG2050) were sterilised by UV in a laminar flow and the culture medium (LONZA EGM-2; Single Quots + 15 ml FCS (fetal calf serum)) was sterile filtrated. The PEG bars were casted in a fibrin clot (2.5 mg/ml fibrinogen, respectively 5 mg/ml fibrinogen; 0.2 UI/ml thrombin). Outgrowth endothelial cells (passage 9) were detached from the bottom of the culture flask by trypsin and diluted in 700 µl culture medium. 10 µl cell suspensions were diluted 1:10 and counted (1,375,000 cells/ml, 962,500 total in 700 µl) and 100 µl (137,500 cells) were injected in the channel and the channel was closed with high concentrated fibrin clots (50 mg/ml fibrinogen, 2 UI/ml thrombin). A 1:30 dilution of an aprotinin stock solution of 3000 KIU/mL was prepared. The clots were incubated (37°C, 5 % CO₂) in 6-well plates with 6 ml medium and 200 µl aprotinin (100 U/ml) for 5 days. The medium was changed every 2-3 days. The clots were incubated in EGM-2 and after 7 days the cells were fixed and tissue slices were subjected to immunohistochemistry with an antibody against CD31 (respectively platelet endothelial cell adhesion molecule; PECAM-1) which is a protein that can be used to identify endothelial cells (Figure 3, left panel). The second antibody is linked with a peroxidase and bound on the first antibody to couple an enzyme to the CD31-antibody-complex. The coupled enzyme is then used to make the complex and the endothelial cells visible by producing a coloured substance. Moreover, staining with an anti-caspase-3 antibody revealed the absence of apoptotic endothelial cells (Figure 3, right panel).

### 4) Provision of longitudinal members of PEG and networks thereof "man-made")

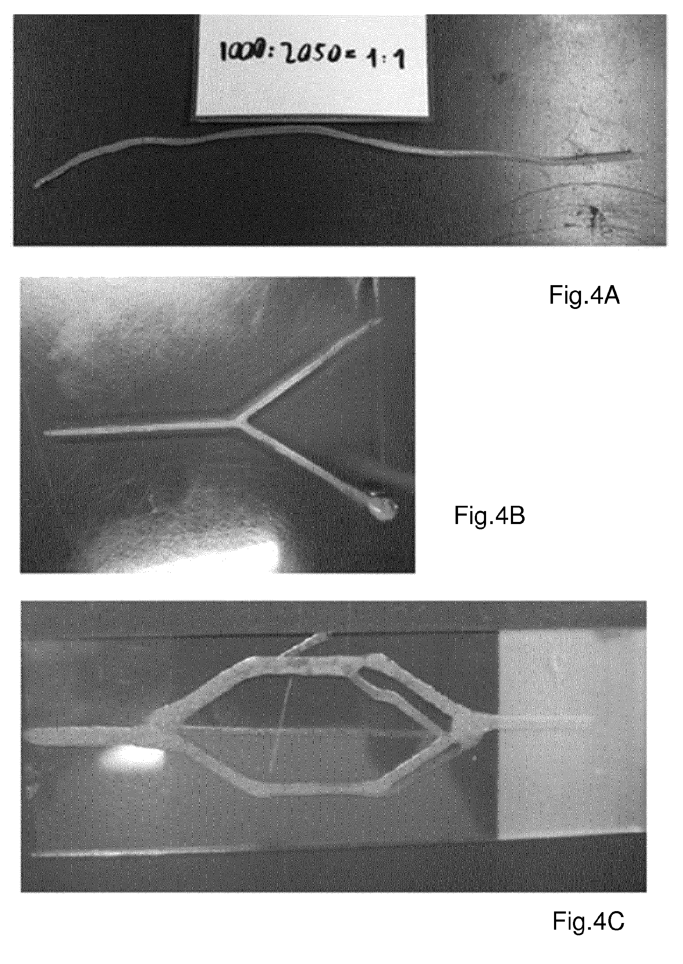

A longitudinal member (Fig. 4A), a Y-shaped branch (Fig. 4B) and a more complex structure (Fig. 4C) were provided by production in silicone tubes (Fig. 4A) or by 3D printing (Figs. 4B and 4C) by using a 50:50 (Fig. 4A) or 12.5:87.5 (Figs. 4B and 4C) mixture of PEG 1000 and PEG 2050. A 3D printer (Fab@Home: Model 2) was assembled from a kit to print the structures applying PEG as printing material. In Figs. 4B and 4C, the PEG mixture was printed into a fibrinogen/thrombin mixture on a glass slide with a printing temperature of 57°C.

### 5) Ingrowth of endothelial cells in the surrounding matrix and formation of tube-like structures in co-culture with adipose-derived stem cells embedded in fibrin ("cell-made").

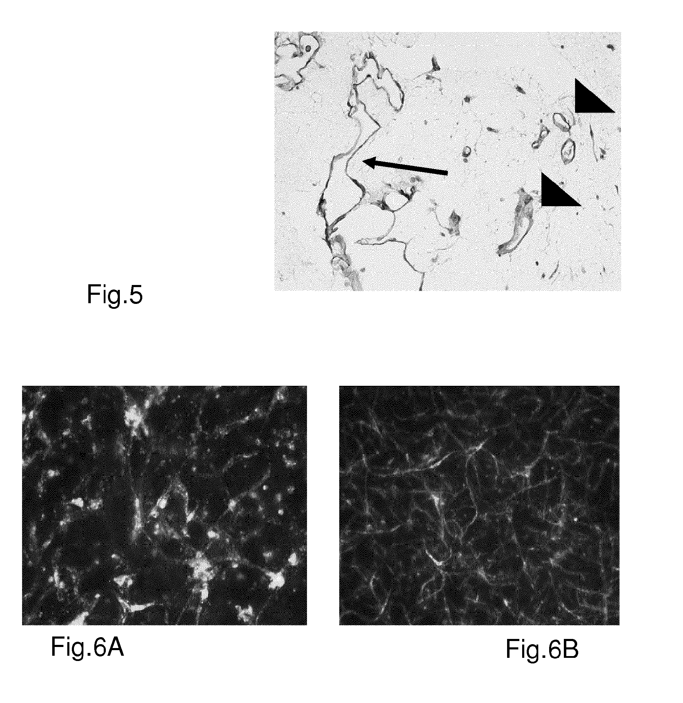

Figure 6 shows outgrowth endothelial cells (OEC) and adipose-derived stem cells co-cultured in a fibrin hydrogel (right panel; B). One week later, formation of tube-like structures is visible, as evidenced by immunofluorescence with anti-CD31 antibody. OEC alone did not show tube forming capability in the fibrin matrix (left panel; A).

In the present examples, the suitability of polyethylene glycol (PEG) to fabricate channels for prevascularisation in fibrin was demonstrated. A spacer formed with PEG was embedded into a fibrin matrix. After the automatic dissolution of the spacer the resulting channel was seeded with OECs and ingrowth of endothelial cells into the surrounding matrix was shown.

### 6) Dissolution properties of PEG and sugar threads

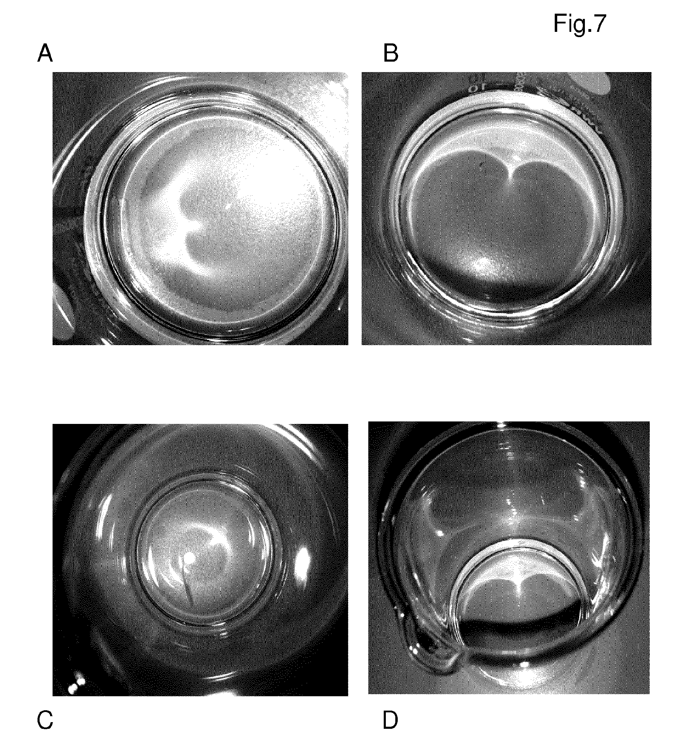

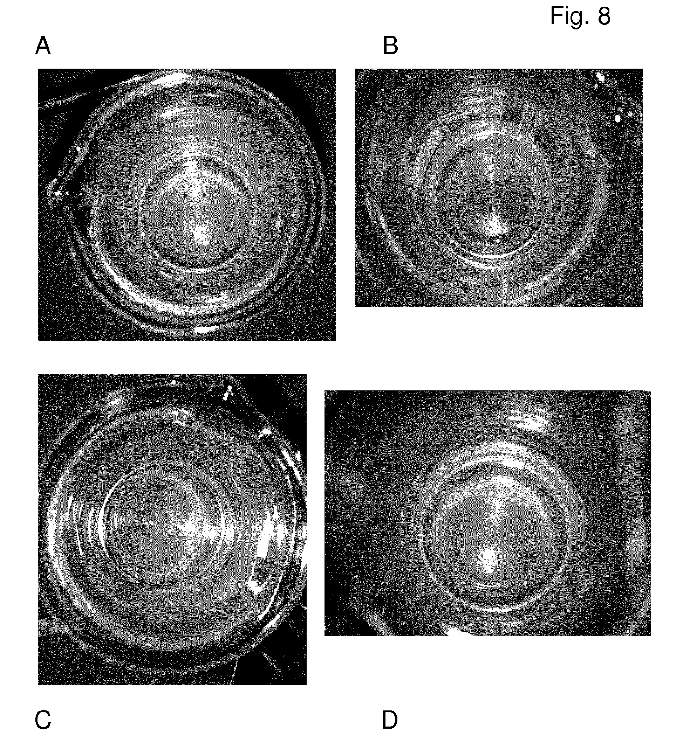

The dissolution properties of PEG and sugar threads in solution was investigated by dissolving thick and thin PEG threads and sugar threads in 10mL NaCl 0.9% prewarmed to 37°C. A tube with 1.5mm inner diameter was cut in half and melted PEG (1g PEG1000 + 7g PEG2000) or melted sucrose were pressed into the tube form. A scalpel was used to get the threads out of the tubing. The longitudinal members obtained ("threads") were about 1.2 to 1.0 cm in length. The results are shown in Figs. 7 and 8. The threads were dissolved immediately within 5 min (PEG) and 6 min (sucrose).

### 7) Results and Discussion:

Vascular tissue engineering aims at mimicking the complexity of the vascular network in cell based scaffolds and promoting angiogenesis in regenerating tissues. Providing sufficient blood supply for complex tissue-engineered constructs is the goal of different prevascularisation strategies. The establishment of a capillary network prior to scaffold implantation has been shown to enhance tissue survival. Importantly, microvessel formation is dependent on the matrix structure the cells are seeded on. In the present examples, the suitability of polyethylene glycol (PEG) to fabricate channels for prevascularisation in fibrin was demonstrated. A spacer formed with PEG was embedded into a fibrin matrix. After dissolution of the spacer the resulting channel was seeded with OECs and ingrowth of endothelial cells into the surrounding matrix was shown. Co-culture with adipose-derived stem cells embedded in fibrin and exposure to shear stress enhanced migration and tube formation of OEC after seven days, was evidenced by immunohistochemical stainings against endothelial cell specific marker CD31 and pericyte marker α-smooth muscle actin. PEG itself does not have an influence on migration and tube formation, as shown by appropriate controls. The present findings demonstrate that PEG is a suitable biomaterial used for applications in vascular tissue engineering.

## Claims

1. Method for producing a hydrogel with one or more channels or a network of channels, said channels having a diameter of 1 cm to 50 µm, wherein one or more longitudinal members or a network of longitudinal members of a first material, said first material having a solubility in water at 25°C of 5 to 500 g/l, is provided and a hydrogel of a second material is formed which hydrogel covers and includes the longitudinal members at least partially and wherein the longitudinal member(s) is (are) at least partially removed from the hydrogel in the course of the formation of the hydrogel to form a hydrogel with one or more channels or a network of channels.

2. Method according to claim 1 or 2, wherein the first material is selected from polyethyleneglycol (PEG), sugars, preferably saccharose, or mixtures thereof.

3. Method according to claim 1 or 2, wherein the hydrogel is formed of a gelation agent selected from fibrinogen, gelatin, chitosan, dextran, PEG, especially crosslinked PEG, polyacrylamide, hydroxypropyl methylcellulose, hydroxypropylcellulose, hydroxymethylcellulose, hydroxyethylcellulose, hydroxypropyl ethylcellulose, methylcellulose, ethylcellulose, carboxyethylcellulose, carboxymethyl hydroxyethylcellulose, carbomer, sodium carboxymethylcellulose, polyvinylpyrrolidone, or mixtures thereof, especially fibrinogen.

4. Method according to any one of claims 1 to 3, wherein the hydrogel contains cells, especially human stem cells, human endothelial cells, fibroblasts, especially dermal fibroblasts, or mixtures thereof.

5. Method according to any one of claims 1 to 4, wherein the longitudinal members have a diameter from 50 µm to 3 cm, preferably from 0.1 to 5 mm, especially from 0.2 to 1 mm.

6. Method according to any one of claims 1 to 5, wherein the longitudinal members have a length of at least four times of the diameter, especially at least six times of the diameter, especially at least ten times of the diameter.

7. Method according to any one of claims 1 to 6, wherein a network of longitudinal members is provided which has T- and/or Y-shaped connections.

8. Method according to any one of claims 1 to 7, wherein the hydrogel contains adipose-derived stem cells (ASCs).

9. Method according to any one of claims 1 to 4, wherein the longitudinal members are sterilised longitudinal members.

10. Method according to any one of claims 1 to 4, wherein the hydrogel is formed out of a solution or suspension of a sterilised hydrogel forming material.

11. Hydrogel with one or more channels or a network of channels obtainable by a method according to any one of claims 1 to 10.

12. Hydrogel according to claim 11 which is sterile and preferably packaged in a pharmaceutically acceptable package.

13. Hydrogel according to claim 11 or 12, further containing cells for promoting vascularisation, preferably human stem cells, human endothelial cells, fibroblasts, especially dermal fibroblasts, or mixtures thereof.

14. Hydrogel according to any one of claims 11 to 13, wherein the first material is PEG, preferably a PEG with a molecular weight of 500 to 2500, especially a mixture of PEGs with a molecular weight of 500 to 2500.

15. Hydrogel according to any one of claims 11 to 14, wherein the hydrogel is formed of fibrinogen, preferably by formation of a fibrin hydrogel by contacting fibrinogen with thrombin.
